(19) Europäisches Patentamt
European Patent Office
Office européen des brevets

(11) EP 4 169 896 A1

(12) EUROPEAN PATENT APPLICATION

(43) Date of publication:
**26.04.2023 Bulletin 2023/17**

(21) Application number: **21203992.9**

(22) Date of filing: **21.10.2021**

(51) International Patent Classification (IPC):
***C07C 13/20*** (2006.01) ***C07C 51/41*** (2006.01)
***C08J 3/20*** (2006.01) ***C08K 5/00*** (2006.01)
***C08K 5/098*** (2006.01)

(52) Cooperative Patent Classification (CPC):
(C-Sets available)
**C08K 5/0083; C07C 51/412; C08J 3/20;**
**C08K 5/098;** C07C 2601/16; C08J 2323/12;
C08K 2201/003; C08L 2205/04; C08L 2207/10

(Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Borealis AG**
**1020 Vienna (AT)**

(72) Inventors:
- **WANG, Jingbo**
  **4021 Linz (AT)**
- **GAHLEITNER, Markus**
  **4021 Linz (AT)**
- **BERNREITNER, Klaus**
  **4021 Linz (AT)**
- **PAN, Cheng**
  **4021 Linz (AT)**

(74) Representative: **Maiwald GmbH**
**Elisenhof**
**Elisenstraße 3**
**80335 München (DE)**

# (54) A BIFUNCTIONAL BETA-NUCLEATING AGENT/FILLER, GENERATED FROM THE SURFACE TREATMENT OF CALCIUM CARBONATE WITH DICARBOXYLIC ACIDS

(57) A process for producing a β-nucleating agent that involves the step of reacting solid calcium carbonate with a dicarboxylic acid according to formula (I):

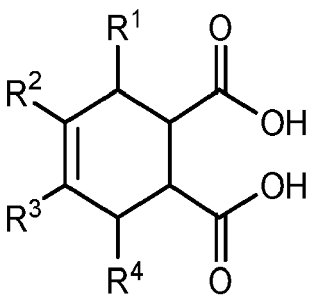

(I)

wherein $R^1$ to $R^4$ are independently selected from the group consisting of hydrogen, alkyl, alkenyl, alkynyl, hydroxyalkyl, cycloalkyl, cycloalkenyl, aryl, substituted aryl, and halide and combinations thereof and optionally any adjacent $R^1$ to $R^4$ are linked together to form a 5-membered or 6-membered ring.

EP 4 169 896 A1

(52) Cooperative Patent Classification (CPC): (Cont.)

C-Sets
**C07C 51/412, C07C 62/30;**
**C08K 5/098, C08L 23/10**

## Description

### Field of the Invention

The present invention is directed to a process for producing a β-nucleating agent from a dicarboxylic acid and calcium carbonate, the β-nucleating agent obtainable by such a process, a polypropylene composition comprising the β-nucleating agent, a process for producing the polypropylene composition, an article comprising the polypropylene composition, and a use of the β-nucleating agent for improving the impact strength of polypropylene compositions.

### Background to the Invention

When cooling from a melt, polypropylene typically crystalizes into the monoclinic α-crystalline form. In addition to this α-form, polypropylene may also crystalize in the hexagonal β-crystalline form and the orthorhombic γ-crystalline form. The metastable β-form is typically characterised by improved impact strength and crack growth retention, which is advantageous for a number of applications, such as in pipes and fittings, but also in profiles and ducts, automotive parts and other technical articles.

Typically, β-crystallisation is achieved through the addition of specific β-nucleating agents, such quinacridone pigments (e.g. in EP 0 177 961 A2), amide compounds (e.g. NJstar NU-100, N,N'-dicyclohexyl-2,6-naphthalene dicarboxamide) and, more recently, heteronuclear rare earth complexes such as WBG (as originally disclosed in Xiao W. et al, J. Appl. Polym. Sci., 111, 1076-1085 (2009)). It is also known that calcium salts of certain simply dibasic acids (such as pimelic acid and suberic acid, as in WO 2008/074494 A1) can perform well as β-nucleating agents, though these salts can be very sensitive to traces of water and other difficult to control parameters.

Whilst a number of β-nucleating agents are known in the art, they are somewhat unreliable when used in industrial processes. Quinacridone pigments furthermore introduce colour to polymer compositions, which is often not desired, depending on the end use of the composition.

As such, further β-nucleating agents having high efficiency, reliability and avoiding pigmentation are desired in the field of polypropylene development.

### Summary of the Invention

The present invention is based on the observation that an efficient bifunctional β-nucleating agent/filler can be generated from the surface treatment of calcium carbonate with certain dicarboxylic acids.

In a first aspect, the present invention is thus directed to a process for producing a β-nucleating agent that involves the step of reacting solid calcium carbonate with a dicarboxylic acid according to formula (I):

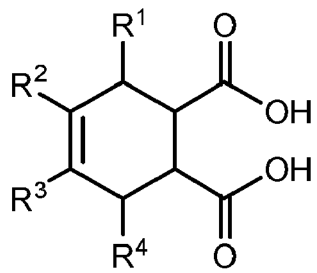

(I)

wherein $R^1$ to $R^4$ are independently selected from the group consisting of hydrogen, alkyl, alkenyl, alkynyl, hydroxyalkyl, cycloalkyl, cycloalkenyl, aryl, substituted aryl, and halide and combinations thereof and optionally any adjacent $R^1$ to $R^4$ are linked together to form a 5-membered or 6-membered ring.

In another aspect, the present invention is directed to a β-nucleating agent obtainable via, more preferably obtained via, the inventive process, wherein the β-nucleating agent essentially consists of calcium carbonate ($CaCO_3$) and the calcium salt of the dicarboxylic acid according to formula (I) (CaCHA) in a weight ratio ([$CaCO_3$]/[CaCHA]) in the range from 15 to 820, more preferably in the range from 20 to 270, most preferably in the range from 26 to 160.

In a further aspect, the present invention is directed to a polypropylene composition (PC) comprising:

i) from 90.0 to 99.9 wt.-%, relative to the total weight of the polypropylene composition, of a propylene polymer (PP); and

ii) from 0.10 to 10.0 wt.-%, relative to the total weight of the polypropylene composition, of the inventive β-nucleating

agent.

In yet another aspect, the present invention is directed to a process for producing the polypropylene composition (PC) of the invention, comprising the steps of:

a) providing the propylene polymer (PP);
b) providing the inventive β-nucleating agent, preferably via the steps of the process described above; and
c) blending and extruding the propylene polymer (PP) and the β-nucleating agent at a temperature in the range from 120 to 250°C in an extruder, preferably a twin-screw extruder, thereby generating the polypropylene composition (PC), preferably in pellet form.

In a final aspect, the present invention is directed to a use of the inventive β-nucleating agent for improving the Charpy notched impact strength of a polypropylene composition comprising 0.10 to 1.00 wt.-% of the β-nucleating agent, wherein the Charpy notched impact strength of the polypropylene composition is in the range from 5.0 to 500%, more preferably by 10.0 to 300%, most preferably from 35 to 200% higher than the Charpy notched impact strength of an equivalent polypropylene composition without the β-nucleating agent, wherein the Charpy notched impact strength is determined at +23 °C according to ISO 179/1eA using 80x10x4 $mm^3$ test bars injection moulded in line with ISO 19069-2.

**Definitions**

Unless defined otherwise, all technical and scientific terms used herein have the same meaning as commonly understood by one of ordinary skill in the art to which the invention pertains. Although, any methods and materials similar or equivalent to those described herein can be used in practice for testing of the present invention, the preferred materials and methods are described herein. In describing and claiming the present invention, the following terminology will be used in accordance with the definitions set out below.

Unless clearly indicated otherwise, use of the terms "a," "an," and the like refers to one or more.

According to the present invention, the expression "propylene homopolymer" relates to a polypropylene that consists substantially, i.e. of at least 99.5 mol-%, more preferably of at least 99.8 mol-%, like of at least 99.9 mol-%, of propylene units. In another embodiment, only propylene units are detectable, i.e. only propylene has been polymerized.

A propylene random copolymer is a copolymer of propylene monomer units and comonomer units, preferably selected from ethylene and C4-C12 alpha-olefins, in which the comonomer units are distributed randomly over the polymeric chain. The propylene random copolymer can comprise comonomer units from one or more comonomers different in their amounts of carbon atoms. In the following amounts are given in mol-% unless it is stated otherwise. A propylene random copolymer must contain at least 50 mol-% propylene units.

Typical for propylene homopolymers and propylene random copolymers is the presence of only one glass transition temperature.

**Detailed Description**

**The process for producing the β-nucleating agent**

The process of the present invention involves the step of reacting solid calcium carbonate with a dicarboxylic acid according to formula (I):

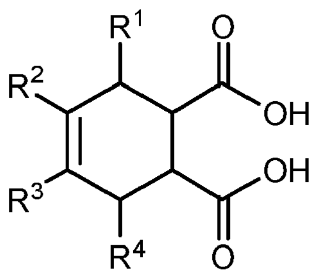

(I)

wherein $R^1$ to $R^4$ are independently selected from the group consisting of hydrogen, alkyl, alkenyl, alkynyl, hydroxyalkyl, cycloalkyl, cycloalkenyl, aryl, substituted aryl, and halide and combinations thereof and optionally any adjacent $R^1$ to $R^4$ are linked together to form a 5-membered or 6-membered ring.

$R^1$ to $R^4$ are preferably independently selected from the group consisting of hydrogen and $C_1$ to $C_4$ alkyl, more preferably from hydrogen, methyl and ethyl.

It is particularly preferred that each of $R^1$ to $R^4$ are hydrogen, i.e. that the dicarboxylic acid is 4-cyclohexene-1,2-dicarboxylic acid, with the most preferred dicarboxylic acid being cis-4-cyclohexene-1,2-dicarboxylic acid.

The calcium carbonate used in the process is preferably in the form of a powder with a median particle diameter (d50) in the range from 1.0 to 20 μm, more preferably in the range from 2.0 to 15 μm, most preferably in the range from 3.0 to 10 μm.

The calcium carbonate may be provided in the form of either ground calcium carbonate (GCC) or precipitated calcium carbonate (PCC). It is preferred that the calcium carbonate is provided in the form of ground calcium carbonate (GCC).

Furthermore, it is preferred that the dicarboxylic acid according to formula (I) is used in an amount of 0.1 to 5.0 wt.-%, more preferably in the range from 0.3 to 4.0 wt.-%, most preferably in the range from 0.5 to 3.0 wt.-%, relative to the weight of the calcium carbonate (i.e. 100 wt.-% $CaCO_3$).

The step of reacting solid calcium carbonate with a dicarboxylic acid according to formula (I) can be preferably carried out via a wet method or a dry method.

In the wet method, a solution of the dicarboxylic acid according to formula (I) in an organic solvent is added to the solid calcium carbonate, followed by removal of the organic solvent under reduced pressure.

This wet method may comprise the step of adding a solution of the dicarboxylic acid according to formula (I) in an organic solvent to a suspension of the solid calcium carbonate in the same organic solvent, followed by removal of the organic solvent under reduced pressure.

Alternatively, the wet method may comprise the step of adding a solution of the dicarboxylic acid according to formula (I) in an organic solvent to the dry solid calcium carbonate, followed by removal of the organic solvent under reduced pressure.

Suitable organic solvents include mono- and dialcohols, ketones and esters. Among monoalcohols, those with low boiling points below 100°C like methanol, ethanol, n-propanol, isopropanol, and tert-butanol are preferred. Likewise, among ketones, those with low boiling points below 100°C like acetone, butanone and pentanone are preferred. Also likewise, among esters, those with low boiling points below 100°C are preferred, like methyl acetate and ethyl acetate. Especially preferred are methanol, ethanol, acetone and butanone. Most preferred is acetone.

In the dry method, the dicarboxylic acid according to formula (I) and the solid calcium carbonate are co-milled until the reaction between the dicarboxylic acid according to formula (I) and the solid calcium carbonate is complete.

Each of these methods may be used to generate surface-treated calcium carbonate powder, which may be used directly as a β-nucleating agent.

It is preferred that no unreacted dicarboxylic acid according to formula (I) remains following the step of reacting solid calcium carbonate with a dicarboxylic acid according to formula (I).

The absence of free acid may be deduced from the FT-IR spectrum, wherein the carbonyl absorption peak of the free acid, which typically may be found at approximately 1690 $cm^{-1}$ is absent and instead there is a carbonyl peak at approximately 1795 $cm^{-1}$, which corresponds to the calcium salt of the dicarboxylic acid according to formula (I).

It is particularly preferred that the process generates a β-nucleating agent as described below.

## The β-nucleating agent

The β-nucleating agent of the present invention is obtainable via, more preferably obtained via, the process as described above.

The β-nucleating agent essentially consists of calcium carbonate ($CaCO_3$) and the calcium salt of the dicarboxylic acid according to formula (I) (CaCHA) in a weight ratio ([$CaCO_3$]/[CaCHA]) in the range from 15 to 820, more preferably in the range from 20 to 270, most preferably in the range from 26 to 160.

The person skilled in the art would understand that commercially available calcium carbonate and commercially available dicarboxylic acids according to formula (I) are rarely 100% pure, thus minor impurities are to be expected.

In one embodiment, the β-nucleating agent comprises at least 90 wt.-%, more preferably at least 95 wt.-%, yet more preferably at least 98 wt.-% of calcium carbonate ($CaCO_3$) and the calcium salt of the dicarboxylic acid according to formula (I) (CaCHA) in a weight ratio ([$CaCO_3$]/[CaCHA]) in the range from 15 to 820, more preferably in the range from 20 to 270, most preferably in the range from 26 to 160, wherein the β-nucleating agent is free from further β-nucleating agents.

It is preferred that the FT-IR spectrum of the β-nucleating agent does not contain any absorption peaks in the range from 1660 to 1640 $cm^{-1}$, more preferably does not contain any absorption peaks in the range from 1680 to 1620 $cm^{-1}$, most preferably does not contain any absorption peaks in the range from 1700 to 1600 $cm^{-1}$.

Preferably, the only peak observed in the region of 2000 to 1500 $cm^{-1}$ is a single peak in the range from 1820 to 1770 $cm^{-1}$, more preferably in the range from 1810 to 1780 $cm^{-1}$, most preferably in the range from 1800 to 1790 $cm^{-1}$.

**The polypropylene composition (PC)**

The polypropylene composition (PC) of the present invention comprises:

i) from 90.0 to 99.9 wt.-%, relative to the total weight of the polypropylene composition, of a propylene polymer (PP); and
ii) from 0.10 to 10.0 wt.-%, relative to the total weight of the polypropylene composition, of the inventive β-nucleating agent.

In a preferred embodiment, the polypropylene composition (PC) comprises:

i) from 93.0 to 99.9 wt.-%, relative to the total weight of the polypropylene composition, of a propylene polymer (PP); and
ii) from 0.10 to 7.0 wt.-%, relative to the total weight of the polypropylene composition, of the inventive β-nucleating agent.

The propylene polymer (PP) may be a propylene homopolymer (h-PP) or a propylene random copolymer (r-PP), preferably a propylene homopolymer (h-PP).

If the propylene polymer is a random copolymer (r-PP), the comonomer content is preferably in the range from 0.01 to 10.0 mol-%, more preferably 0.05 to 5.0 mol-%, most preferably in the range from 0.10 to 3.0 mol-%.

The comonomer of the propylene random copolymer (r-PP) is preferably selected from ethylene and C4 to C8 alpha olefins, more preferably is ethylene or 1-butene (C4).

If the propylene polymer (PP) is a propylene homopolymer (h-PP), it is preferred that the propylene homopolymer (h-PP) has an isotactic pentad regularity <mmmm> determined by $^{13}C$-NMR spectroscopy in the range of 90.0 to 99.9%, more preferably in the range from 91.0 to 99.5%, most preferably in the range from 92.5 to 98.5%.

It is further preferred that the propylene polymer (PP) has a melt flow rate ($MFR_2$), determined according to ISO 1133 at 230 °C at a load of 2.16 kg, in the range from 0.1 to 5.0 g/10 min, more preferably in the range from 0.1 to 2.0 g/10 min, most preferably in the range from 0.1 to 1.0 g/10 min.

The polypropylene composition (PC) preferably has a melt flow rate ($MFR_2$), determined according to ISO 1133 at 230 °C at a load of 2.16 kg, in the range from 0.1 to 5.0 g/10 min, more preferably in the range from 0.1 to 2.0 g/10 min, most preferably in the range from 0.1 to 1.0 g/10 min.

The polypropylene composition (PC) preferably has a crystallisation temperature ($T_c$), determined by DSC analysis, in the range from 112.0 to 130.0 °C, more preferably in the range from 114.0 to 128.0 °C, most preferably in the range from 115.0 to 126.0 °C.

The polypropylene composition (PC) preferably has a flexural modulus, determined according to ISO 178 using 80x10x4 $mm^3$ test bars injection moulded in line with ISO 19069-2, in the range from 1000 to 1500 MPa, more preferably in the range from 1100 to 1450 MPa, most preferably in the range from 1200 to 1400 MPa.

The polypropylene composition (PC) preferably has a Charpy notched impact strength (NIS), determined at +23 °C according to ISO 179/1eA using 80x10x4 $mm^3$ test bars injection moulded in line with ISO 19069-2, in the range from 5.0 to 50.0 $kJ/m^2$, more preferably in the range from 7.0 to 30.0 $kJ/m^2$, most preferably in the range from 8.0 to 20.0 $kJ/m^2$.

It is particularly preferred that the polypropylene composition (PC) according to any one of the preceding claims, having a first melting temperature ($T_{m1}$) in the range from 157 to 167 °C and a second melting temperature ($T_{m2}$) in the range from 142 to 155 °C, wherein the ratio between the enthalpy of fusion associated with the first melting temperature ($\Delta H_{m1}$) and the enthalpy of fusion associated with the second melting temperature ($\Delta H_{m2}$), ($[\Delta H_{m1}]/[\Delta H_{m2}]$) is in the range from 0.1 to 2.3, more preferably in the range from 0.1 to 1.9, most preferably in the range from 0.1 to 1.5, wherein the first melting temperature ($T_{m1}$), the second melting temperature ($T_{m2}$), the enthalpy of fusion associated with the first melting temperature ($\Delta H_{m1}$) and the enthalpy of fusion associated with the second melting temperature ($\Delta H_{m2}$) are determined according to DSC analysis.

It is further preferred that the first melting temperature ($T_{m1}$) is in the range from 160 to 167 °C, more preferably in the range from 162 to 165 °C.

It is also further preferred that the second melting temperature ($T_{m2}$) is in the range from 144 to 152 °C, more preferably in the range from 146 to 149 °C.

**Article**

In a further aspect, the present invention is directed to an article comprising the polypropylene composition according to the invention.

The article is either an extruded article or a moulded article. Preferably, the article is selected from the group consisting of pipes and fittings, profiles and ducts, automotive parts and technical articles.

The article comprises at least 90 wt.-%, more preferably at least 95 wt.-%, most preferably at least 98 wt.-% of the polypropylene composition (PC) as described above.

It is further preferred that the extruded or moulded article has a core beta-phase content ($K_\beta$), determined by wide angle x-ray scattering (WAXS), in the range from 20 to 99%, more preferably in the range from 30 to 95%, most preferably in the range from 50 to 90%.

Preferably, the extruded or moulded article has a core crystallinity index (Xc) in the range from 50 to 80%, more preferably in the range from 55 to 70%, most preferably in the range from 58 to 63%.

All preferable embodiments and fallback positions for the polypropylene composition (PC) described above apply mutatis mutandis to the polypropylene composition (PC) of the article.

**Process for producing the polypropylene composition (PC)**

In another aspect, the present invention is directed to a process for producing the polypropylene composition (PC) of the invention.

Said process comprises the steps of:

a) providing the propylene polymer (PP);
b) providing the inventive β-nucleating agent, preferably via the steps of the process described above; and
c) blending and extruding the propylene polymer (PP) and the β-nucleating agent at a temperature in the range from 120 to 250 °C in an extruder, preferably a twin-screw extruder, thereby generating the polypropylene composition (PC), preferably in pellet form.

In particular, it is preferred to use a conventional compounding or blending apparatus, e.g. a Banbury mixer, a 2-roll rubber mill, Buss-co-kneader or a twin-screw extruder. More preferably, mixing is accomplished in a co-rotating twin-screw extruder. The polymer materials recovered from the extruder are usually in the form of pellets.

It is particularly preferred that the polypropylene composition (PC) of the present invention is used for the production of moulded articles. It is thus preferred that the process further comprises, after step c) the step of:
d) moulding the polypropylene composition (PC) produced in step c) to form a moulded article, wherein the moulding is preferably injection moulding.

Alternatively, the polypropylene composition (PC) of the present invention is used for the production of extruded articles. In such embodiments, step c) may involve:
c) blending and extruding the propylene polymer (PP) and the β-nucleating agent at a temperature in the range from 120 to 250 °C in an extruder, preferably a twin-screw extruder, thereby generating an extruded article comprising the polypropylene composition (PC).

All preferable embodiments and fallback positions for the polypropylene composition (PC) and/or the article described above apply mutatis mutandis to the process for producing the polypropylene composition (PC) and/or the article.

**Use**

In a final aspect, the present invention is directed to the inventive β-nucleating agent for improving the Charpy notched impact strength of a polypropylene composition comprising 0.10 to 10.0 wt.-% of the inventive β-nucleating agent.

The improvement of the Charpy notched impact strength is achieved when the Charpy notched impact strength of the polypropylene composition is in the range from 5.0 to 500%, more preferably by 10.0 to 300%, most preferably from 35 to 200% higher than the Charpy notched impact strength of an equivalent polypropylene composition without the β-nucleating agent, wherein the Charpy notched impact strength is determined at +23 °C according to ISO 179/1eA using 80x10x4 $mm^3$ test bars injection moulded in line with ISO 19069-2.

In a preferred embodiment, the polypropylene composition of the use described above is the polypropylene composition (PC) as described above.

All preferable embodiments and fallback positions for the β-nucleating agent and the polypropylene composition (PC) described above apply mutatis mutandis to the β-nucleating agent and the polypropylene composition (PC) of this embodiment.

## EXAMPLES

### A. Measuring methods

The following definitions of terms and determination methods apply for the above general description of the invention including the claims as well as to the below examples unless otherwise defined.

### Quantification of microstructure by NMR spectroscopy

Quantitative nuclear-magnetic resonance (NMR) spectroscopy was used to quantify the isotacticity and regio-regularity of the propylene homopolymers.

Quantitative $^{13}C\{^1H\}$ NMR spectra were recorded in the solution-state using a Bruker Advance III 400 NMR spectrometer operating at 400.15 and 100.62 MHz for $^1H$ and $^{13}C$ respectively. All spectra were recorded using a $^{13}C$ optimised 10 mm extended temperature probehead at 125°C using nitrogen gas for all pneumatics.

For propylene homopolymers approximately 200 mg of material was dissolved in 1,2-tetrachloroethane-$d_2$ (TCE-$d_2$). To ensure a homogenous solution, after initial sample preparation in a heat block, the NMR tube was further heated in a rotatary oven for at least 1 hour. Upon insertion into the magnet the tube was spun at 10 Hz. This setup was chosen primarily for the high resolution needed fortacticity distribution quantification (Busico, V., Cipullo, R., Prog. Polym. Sci. 26 (2001) 443; Busico, V.; Cipullo, R., Monaco, G., Vacatello, M., Segre, A.L., Macromolecules 30 (1997) 6251). Standard single-pulse excitation was employed utilising the NOE and bi-level WALTZ 16 decoupling scheme (Zhou, Z., Kuemmerle, R., Qiu, X., Redwine, D., Cong, R., Taha, A., Baugh, D. Winniford, B., J. Mag. Reson. 187 (2007) 225; Busico, V., Carbonniere, P., Cipullo, R., Pellecchia, R., Severn, J., Talarico, G., Macromol. Rapid Commun. 2007, 28, 11289). A total of 8192 (8k) transients were acquired per spectra.

Quantitative $^{13}C\{^1H\}$ NMR spectra were processed, integrated and relevant quantitative properties determined from the integrals using proprietary computer programs.

For propylene homopolymers all chemical shifts are internally referenced to the methyl isotactic pentad (mmmm) at 21.85 ppm.

Characteristic signals corresponding to regio defects (Resconi, L., Cavallo, L., Fait, A., Piemontesi, F., Chem. Rev. 2000, 100, 1253; Wang, W-J., Zhu, S., Macromolecules 33 (2000), 1157; Cheng, H. N., Macromolecules 17 (1984), 1950) or comonomer were observed.

The tacticity distribution was quantified through integration of the methyl region between 23.6-19.7 ppm correcting for any sites not related to the stereo sequences of interest (Busico, V., Cipullo, R., Prog. Polym. Sci. 26 (2001) 443; Busico, V., Cipullo, R., Monaco, G., Vacatello, M., Segre, A.L., Macromolecules 30 (1997) 6251).

Specifically the influence of regio-defects and comonomer on the quantification of the tacticity distribution was corrected for by subtraction of representative regio-defect and comonomer integrals from the specific integral regions of the stereo sequences.

The isotacticity was determined at the pentad level and reported as the percentage of isotactic pentad (mmmm) sequences with respect to all pentad sequences:

$$[mmmm]\ \% = 100 * (mmmm / \text{sum of all pentads})$$

The presence of 2,1 erythro regio-defects was indicated by the presence of the two methyl sites at 17.7 and 17.2 ppm and confirmed by other characteristic sites. Characteristic signals corresponding to other types of regio-defects were not observed (Resconi, L., Cavallo, L., Fait, A., Piemontesi, F., Chem. Rev. 2000, 100, 1253).

The amount of 2,1 erythro regio-defects was quantified using the average integral of the two characteristic methyl sites at 17.7 and 17.2 ppm:

$$P_{21e} = (I_{e6} + I_{e8}) / 2$$

The amount of 1,2 primary inserted propylene was quantified based on the methyl region with correction undertaken for sites included in this region not related to primary insertion and for primary insertion sites excluded from this region:

$$P_{12} = I_{CH3} + P_{12e}$$

The total amount of propylene was quantified as the sum of primary inserted propylene and all other present regio-defects:

$$P_{total} = P_{12} + P_{21e}$$

The mole percent of 2,1 erythro regio-defects was quantified with respect to all propylene:

$$[21e]\ \text{mol-\%} = 100 * (P_{21e} / P_{total})$$

**$MFR_2$ (230 °C)** was measured according to ISO 1133 (230 °C, 2.16 kg load).

**DSC analysis, melting temperature ($T_m$) and heat of fusion ($H_f$), crystallization temperature ($T_c$) and heat of crystallization ($H_c$):** measured with a TA Instrument Q200 differential scanning calorimetry (DSC) on 5 to 7 mg samples. DSC is run according to ISO 11357 / part 3 /method C2 in a heat / cool / heat cycle with a scan rate of 10 °C/min in the temperature range of -30 to +225°C. Crystallization temperature ($T_c$) and heat of crystallization ($H_c$) are determined from the cooling step, while melting temperature ($T_m$) and heat of fusion ($H_f$) are determined from the second heating step.

**The Flexural Modulus** is determined according to ISO 178 method A (3-point bending test) on 80×10×4 $mm^3$ specimens. Following the standard, a test speed of 2 mm/min and a span length of 16 times the thickness was used. The testing temperature was 23±2° C. Injection moulding was carried out according to ISO 19069-2 using a melt temperature of 230°C for all materials irrespective of material melt flow rate.

**Notched impact strength (NIS)**

The Charpy notched impact strength (NIS) was measured according to ISO 179 1eA at +23°C or -20 °C, using injection moulded bar test specimens of 80x10x4 $mm^3$ specimens. Injection moulding was carried out according to ISO 19069-2 using a melt temperature of 230°C for all materials irrespective of material melt flow rate.

**Wide angle X-ray Scattering measurement (WAXS)**

The measurement of wide-angle X-ray scattering (WAXS) of the samples was conducted by a Bruker D8 Discover apparatus. The diffractometer was equipped with an X-ray tube with a copper target operating at 30 kV and 20mA and a GADDS 2-D detector. A point collimation (0.5 mm) was used to direct the beam onto the surface. The measurement was done in reflection geometry, and 28 angle in the range from 10° to 32.5° were measured. Data were collected for 300 s. Intensity vs. 2-theta curve was acquired with the same measurement parameters on an amorphous polypropylene sample, which was prepared by solvent extraction. An amorphous halo was obtained by smoothing the curve. The amorphous halo has been subtracted from the measured intensity vs. 2-theta curve to result in the crystalline curve.

The crystallinity index Xc can be defined by the area under the crystalline curve and the original spectrum using Challa, Hermans and Weidinger method [Challa F, Hermans PH, Weidinger A, Makromol. Chem. 56, 169 (1962)] as:

$$Xc = \frac{area\ under\ crystalline\ curve}{area\ under\ original\ spectrum} \times 100$$

The amount of β-form of the polypropylene within the crystalline phase $K_\beta$ is calculated using Jones method [Turner-Jones A, Aizlewood JM, Beckett DR, Makromol. Chem. 75, 134 (1974)] according to the following equation:

$$K_\beta = \frac{I^\beta(300)}{I^\alpha(110) + I^\alpha(040) + I^\alpha(130) + I^\beta(300)}$$

where, $I^\beta(300)$ is the intensity of β(300) peak, $I^\alpha$ (1 10) is the intensity of α(1 10) peak, $I^\alpha$ (040) is the intensity of α(040) peak and $I^\alpha$ (130) is the intensity of α(130) peak obtained after subtracting the amorphous halo.

The amount of γ-form of isotactic polypropylene (iPP) within the crystalline phase $K_\gamma$ is calculated using the method developed by Pae [Pae KD, J. Polym. Sci. , Part A, 6, 657 (1968)] as:

$$K_{\gamma} = \frac{I^{\gamma}(117)}{I^{\alpha}(130) + I^{\gamma}(117)}$$

where, $I^{\alpha}$ (130) is the intensity of $\alpha$(130) peak and $I^{\gamma}$ (117) is the intensity of $\gamma$(117) peak obtained after subtracting a base line joining the base of these peaks.

Quantification of three-phase crystalline system has been carried out following the procedure explained in Obadal M, Cermak R, Stoklasa K, Macromol. Rapid Commun. 26, 1253 (2005). For three-phase crystalline systems the following equations have been used to determine $K_{\alpha}$ (amount of $\alpha$-phase), $K_{\beta}$ (amount of $\beta$-phase) and $K\gamma$ (amount of $\gamma$-phase):

$$K_{\beta} = \frac{I^{\beta}(300)}{I^{\alpha}(110) + I^{\alpha}(040) + I^{\alpha}(130) + I^{\beta}(300) + I^{\gamma}(117)}$$

$$K_{\alpha+\gamma} = 1 - K_{\beta}$$

$$K_{\gamma} = G \times K_{\alpha+\gamma}$$

and

$$K_{\alpha} = 1 - K_{\beta} - K_{\gamma}$$

Measurements were performed on the skin section (i.e. the outer 100 $\mu$m) and the core section (i.e. the central part) of 80x10x4 $mm^3$ specimens as injection molded for the mechanical tests.

**Fourier Transform infrared spectroscopy (FT-IR)**

The initial components, calcium carbonate and cis-4-cyclohexene-1,2-dicarboxylic acid, as well as the reaction products of the different routes, NU1 to NU3, were characterized in a Bruker Tensor 27 FT-IR spectrometer. Samples were prepared by pressing in a 1:1-mixture with potassium bromide (KBr) and measured in attenuated total reflection (ATR) mode in a wave number range of 4000 to 400 $cm^{-1}$ corresponding to a wavelength of 2.5 to 25 $\mu$m.

**Median Particle Size (*$d_{50}$*)**

The dso was calculated from the particle size distribution as determined by laser diffraction method, using Laser Mastersizer, according to ISO 13320-1. The dso is defined as the median diameter, wherein 50% of the particles have a diameter smaller than the $d_{50}$ value and the other 50% of the particles have a diameter greater than the $d_{50}$ value.

**B. Experimental**

**1. Preparation of $\beta$-nucleating agents**

Three $\beta$-nucleating agents were prepared from cis-4-cyclohexene-1,2-dicarboxylic acid (CHA) (CAS No: 2305-26-2), commercially available from SigmaAldrich, and calcium carbonate ($CaCO_3$), commercially available from Mineraria Sacilese under the trade name Calcitec M/5, which has a median particle diameter d50 of 5.0 $\mu$m and a top-cut diameter d99 of 20 $\mu$m.

**NU1**: 200 g of $CaCO_3$ were dispersed in 250 ml acetone at room temperature, with a magnetic stirrer, 4 g of CHA were dissolved into 30 ml acetone and then the solution was added to the $CaCO_3$ suspension dropwise under stirring. The mixture was stirred at room temperature for extra 20 min to allow the full reaction. The acetone was removed and the resultant powder was used directly.

**NU2:** 0.1 g of CHA and 10 g of $CaCO_3$ were milled with a Ball miller at RT for 10 min. Several repetitions were done to obtain a sufficient amount of the powder, which was used directly.

**NU3:** 4 g of CHA were dissolved into 30 ml acetone and then the solution was spayed to the 200 g $CaCO_3$, then the mixture was mixed intensively. The acetone was removed and the resultant powder was used directly.

As can be seen from Figures 1 to 5, FT-IR spectroscopy indicates that the CHA has fully reacted, with the carbonyl peak at 1690 cm-1 that indicates the presence of CHA (see Figure 1) fully absent in the three β-nucleating agents NU1 to NU3 (Figures 3 to 5), with peaks instead observed at approximately 1795 $cm^{-1}$. Figure 2 shows that $CaCO_3$ has no peaks in this region of the spectrum.

**2. Preparation of polypropylene compositions**

The inventive and comparative examples were compounded in a TSE16 twin screw extruder with a melt temperature of 210 °C and a throughput rate of 1.5 kg/h, according to the recipes given in Table 1:

**Table 1:** Recipes of the inventive and comparative examples

| | | CE1 | CE2 | IE1 | IE2 | IE3 | IE4 | IE5 | IE6 |
|---|---|---|---|---|---|---|---|---|---|
| h-PP1 | [wt%] | 99.55 | 99.80 | 99.60 | 98.80 | 94.80 | 98.80 | 98.80 | 99.30 |
| AO | [wt%] | 0.15 | 0.15 | 0.15 | 0.15 | 0.15 | 0.15 | 0.15 | 0.15 |
| SHT | [wt%] | 0.05 | 0.05 | 0.05 | 0.05 | 0.05 | 0.05 | 0.05 | 0.05 |
| CHA | [wt%] | 0.25 | - | - | - | - | - | - | - |
| NU1 | [wt%] | - | - | 0.20 | 1.00 | 5.00 | - | - | - |
| NU2 | [wt%] | - | - | - | - | - | 1.00 | - | - |
| NU3 | [wt%] | - | - | - | - | - | - | 1.00 | 0.50 |

h-PP1 a propylene homopolymer having an $MFR_2$ of 0.7 g/10 min and an isotactic pentad concentration of 93.1%, commercially available from Borealis AG under the trade name HA001-A.
AO Irganox B215, a synergistic 2:1 blend of antioxidants Irgafos 168 (tris(2,4-ditert-butylphenyl)phosphite, CAS No: 31570-04-4) and Irganox 1010 (pentaerythritol tetrakis[3-[3,5-di-tert-butyl-4-hydroxyphenyl]propionate], CAS No: 6683-19-8), available from BASF SE.
SHT synthetic hydrotalcite, available from Kisuma Chemicals under the trade name DHT-4A.
CHA cis-4-cyclohexene-1,2-dicarboxylic acid (CAS No: 2305-26-2), commercially available from SigmaAldrich.

**Table 2:** Properties of the inventive and comparative examples

| | | CE1 | CE2 | IE1 | 1E2 | IE3 | IE4 | IE5 | IE6 |
|---|---|---|---|---|---|---|---|---|---|
| $MFR_2$ | [g/10min] | 0.75 | 0.63 | 0.53 | 0.54 | 0.56 | 0.50 | 0.56 | 0.65 |
| Flex Mod | [MPa] | 1539 | 1404 | 1317 | 1328 | 1336 | 1342 | 1361 | 1372 |
| NIS | [$kJ/m^2$] | 5.52 | 5.37 | 10.53 | 8.19 | 8.95 | 7.03 | 5.72 | 5.62 |
| ΔNIS | [%] | 3 | n/a | 96 | 53 | 67 | 31 | 7 | 5 |
| **DSC** | | | | | | | | | |
| Tc | [°C] | 117 | 117 | 116 | 118 | 119 | 117 | 118 | 116 |
| $T_{m1}$ | [°C] | 163 | 163 | 164 | 162 | 162 | 162 | 162 | 163 |
| $H_{m1}$ | [J/g] | 97 | 103 | 45 | 58 | 48 | 62 | 65 | 66 |
| $T_{m2}$ | [°C] | n.d. | n.d. | 148 | 147 | 148 | 147 | 148 | 148 |
| $H_{m2}$ | [J/g] | n.d. | n.d. | 52 | 45 | 52 | 41 | 39 | 36 |
| $[\Delta H_{m1}]/[\Delta H_{m2}]$ | [-] | n/a | n/a | 0.86 | 1.29 | 0.92 | 1.51 | 1.67 | 1.83 |
| **WAXS B-bar** | | | | | | | | | |
| Xc(core) | [%] | 66.7 | 64.9 | 60.4 | 60.9 | 62.3 | 63.5 | 63.9 | 63.9 |
| Kp(core) | [%] | 0.02 | 0.09 | 0.68 | 0.59 | 0.86 | 0.38 | 0.25 | 0.23 |
| Xc (skin) | [%] | 64.9 | 63.2 | 59.4 | 60.5 | 62.9 | 61.4 | 61.9 | 61.8 |

(continued)

| WAXS B-bar | | | | | | | | | |
|---|---|---|---|---|---|---|---|---|---|
| $K_{\beta}$(skin) | [%] | 0.22 | 0.27 | 0.41 | 0.39 | 0.39 | 0.31 | 0.35 | 0.32 |
| n/a - not applicable, n.d. - not detectable (below the detection limit) | | | | | | | | | |

As can be seen from Table 2, each of the β-nucleating agents NU1 to NU3 are efficient β-nucleating agents, with significant β-crystallisation observed by WAXS analysis. The impact strength of the nucleated compositions are notably higher than those of the non-nucleated composition (CE2) and higher than the use of CHA alone (CE1).

**Claims**

1. A process for producing a β-nucleating agent that involves the step of reacting solid calcium carbonate with a dicarboxylic acid according to formula (I):

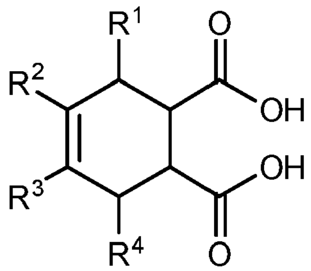

(I)

wherein $R^1$ to $R^4$ are independently selected from the group consisting of hydrogen, alkyl, alkenyl, alkynyl, hydroxyalkyl, cycloalkyl, cycloalkenyl, aryl, substituted aryl, and halide and combinations thereof and optionally any adjacent $R^1$ to $R^4$ are linked together to form a 5-membered or 6-membered ring.

2. The process according to claim 1, wherein $R^1$ to $R^4$ are independently selected from the group consisting of hydrogen and $C_1$ to $C_4$ alkyl, more preferably $R^1$ to $R^4$ are each hydrogen.

3. The process according to claim 1 or claim 2, wherein the calcium carbonate is in the form of a powder with a median particle diameter (dso), as determined by laser diffraction according to ISO 13320-1, in the range from 1.0 to 20 μm, more preferably in the range from 2.0 to 15 μm, most preferably in the range from 3.0 to 10 μm.

4. The process according to any one of the preceding claims, wherein the dicarboxylic acid according to formula (I) is used in an amount of 0.1 to 5.0 wt.-%, more preferably in the range from 0.3 to 4.0 wt.-%, most preferably in the range from 0.5 to 3.0 wt.-%, relative to the weight of the calcium carbonate.

5. The process according to any one of the preceding claims, wherein a solution of the dicarboxylic acid according to formula (I) in an organic solvent is added to the solid calcium carbonate, followed by removal of the organic solvent under reduced pressure.

6. The process according to any one of claims 1 to 4, wherein the dicarboxylic acid according to formula (I) and the solid calcium carbonate are co-milled until the reaction between the dicarboxylic acid according to formula (I) and the solid calcium carbonate is complete.

7. A β-nucleating agent obtainable via, more preferably obtained via, the process according to any one of the preceding claims, wherein the β-nucleating agent essentially consists of calcium carbonate ($CaCO_3$) and the calcium salt of the dicarboxylic acid according to formula (I) (CaCHA) in a weight ratio ([$CaCO_3$]/[CaCHA]) in the range from 15 to 820, more preferably in the range from 20 to 270, most preferably in the range from 26 to 160.

8. A polypropylene composition (PC) comprising:

i) from 90.0 to 99.9 wt.-%, relative to the total weight of the polypropylene composition, of a propylene polymer (PP); and
ii) from 0.10 to 10.0 wt.-%, relative to the total weight of the polypropylene composition, of the β-nucleating agent according to claim 7.

**9.** A polypropylene composition (PC) according to claim 8, wherein the propylene polymer (PP) is a propylene homopolymer (h-PP) having at least one of, preferably both of, the following properties:

a) a melt flow rate ($MFR_2$), determined according to ISO 1133 at 230 °C at a load of 2.16 kg, in the range from 0.1 to 5.0 g/10 min, more preferably in the range from 0.1 to 2.0 g/10 min, most preferably in the range from 0.1 to 1.0 g/10 min; and
b) an isotactic pentad regularity <mmmm> determined by $^{13}C$-NMR spectroscopy in the range of 90.0 to 99.9%, more preferably in the range from 91.0 to 99.5%, most preferably in the range from 92.5 to 98.5%.

**10.** The polypropylene composition (PC) according to claim 8 or claim 9, wherein the polypropylene composition (PC) has at least one of, preferably both of, the following properties:

a) a melt flow rate ($MFR_2$), determined according to ISO 1133 at 230 °C at a load of 2.16 kg, in the range from 0.1 to 5.0 g/10 min, more preferably in the range from 0.1 to 2.0 g/10 min, most preferably in the range from 0.1 to 1.0 g/10 min;
b) a crystallisation temperature ($T_c$), determined by DSC analysis, in the range from 112.0 to 130.0 °C, more preferably in the range from 114.0 to 128.0 °C, most preferably in the range from 115.0 to 126.0 °C;
c) a flexural modulus, determined according to ISO 178 using 80x10x4 $mm^3$ test bars injection moulded in line with ISO 19069-2, in the range from 1000 to 1500 MPa, more preferably in the range from 1100 to 1450 MPa, most preferably in the range from 1200 to 1400 MPa; and
d) a Charpy notched impact strength, determined at +23 °C according to ISO 179/1eA using 80x10x4 $mm^3$ test bars injection moulded in line with ISO 19069-2, in the range from 5.0 to 50.0 $kJ/m^2$, more preferably in the range from 7.0 to 30.0 $kJ/m^2$, most preferably in the range from 8.0 to 20.0 $kJ/m^2$.

**11.** The polypropylene composition (PC) according to any one of claims 8 to 10, having a first melting temperature ($T_{m1}$) in the range from 157 to 167 °C and a second melting temperature (Tm2) in the range from 142 to 155 °C, wherein the ratio between the enthalpy of fusion associated with the first melting temperature ($\Delta H_{m1}$) and the enthalpy of fusion associated with the second melting temperature ($\Delta H_{m2}$), ($[\Delta H_{m1}]/[\Delta H_{m2}]$) is in the range from 0.1 to 2.3, more preferably in the range from 0.1 to 1.9, most preferably in the range from 0.1 to 1.5, wherein the first melting temperature ($T_{m1}$), the second melting temperature ($T_{m2}$), the enthalpy of fusion associated with the first melting temperature ($\Delta H_{m1}$) and the enthalpy of fusion associated with the second melting temperature ($\Delta H_{m2}$) are determined according to DSC analysis.

**12.** A process for producing the polypropylene composition (PC) according to any one of claims 8 to 11, comprising the steps of:

a) providing a propylene polymer (PP) as defined in claim 8 or 9;
b) providing the β-nucleating agent according to claim 7 , preferably via the process according to any one of claims 1 to 6; and
c) blending and extruding the propylene polymer (PP) and the β-nucleating agent at a temperature in the range from 120 to 250 °C in an extruder, preferably a twin-screw extruder, thereby generating the polypropylene composition (PC), preferably in pellet form.

**13.** An article, being either an extruded or a molded article, comprising at least 90 wt.-%, more preferably at least 95 wt.-%, most preferably at least 98 wt.-% of the polypropylene composition (PC) according to any one of claims 8 to 11, preferably having a core beta-phase content, determined by wide angle x-ray scattering (WAXS), in the range from 20 to 99%, more preferably in the range from 30 to 95%, most preferably in the range from 50 to 90%.

**14.** A use of a β-nucleating agent according to claim 7 for improving the Charpy notched impact strength of a polypropylene composition comprising 0.10 to 1.00 wt.-% of the β-nucleating agent, wherein the Charpy notched impact strength of the polypropylene composition is in the range from 5.0 to 500%, more preferably by 10.0 to 300%, most preferably from 35 to 200% higher than the Charpy notched impact strength of an equivalent polypropylene composition without the β-nucleating agent, wherein the Charpy notched impact strength is determined at +23 °C according to ISO 179/1eA using 80x10x4 $mm^3$ test bars injection moulded in line with ISO 19069-2.

**15.** The use according to claim 14, wherein the polypropylene composition is a polypropylene composition (PC) according to any one of claims 8 to 11.

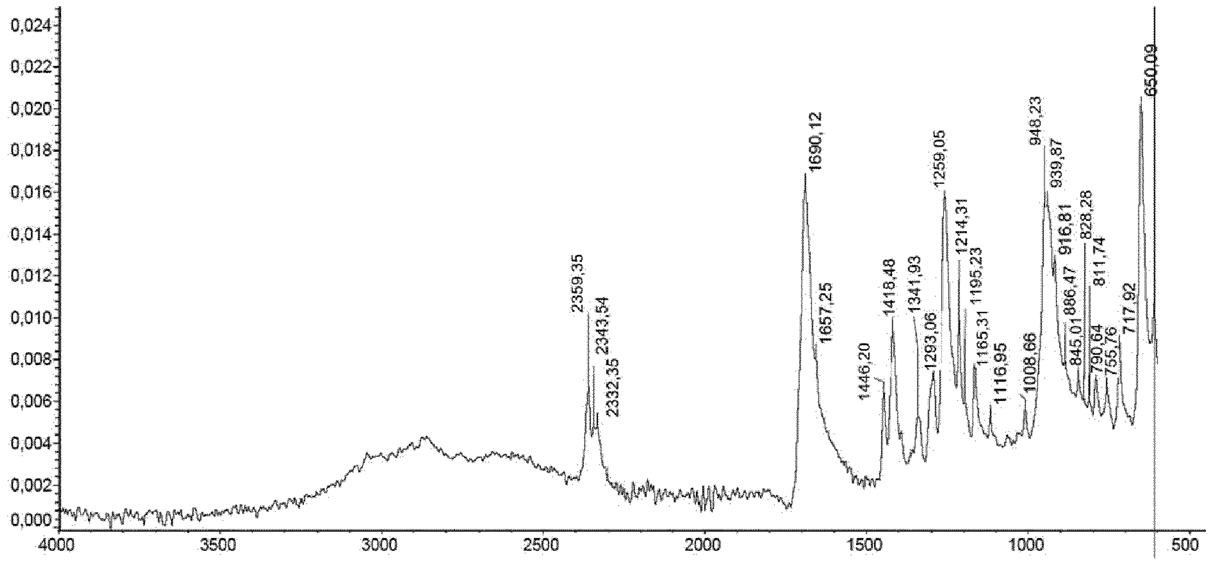


**Figure 1** FT-IR spectrum of cis-4-cyclohexene-1,2-dicarboxylic acid (CHA)

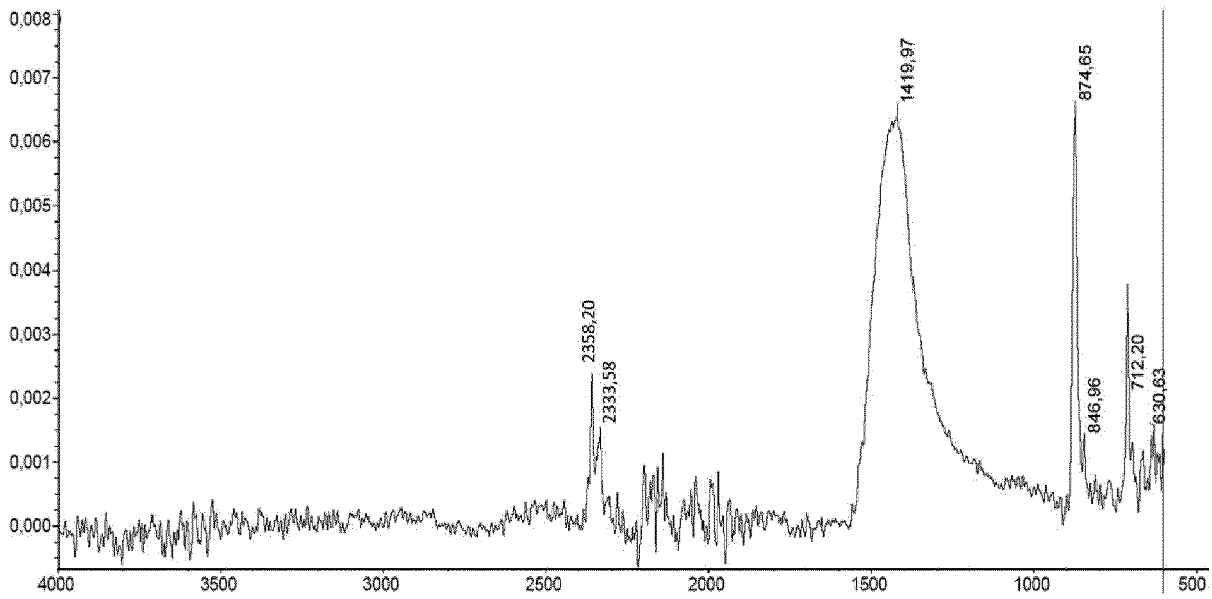


**Figure 2** FT-IR spectrum of $CaCO_3$

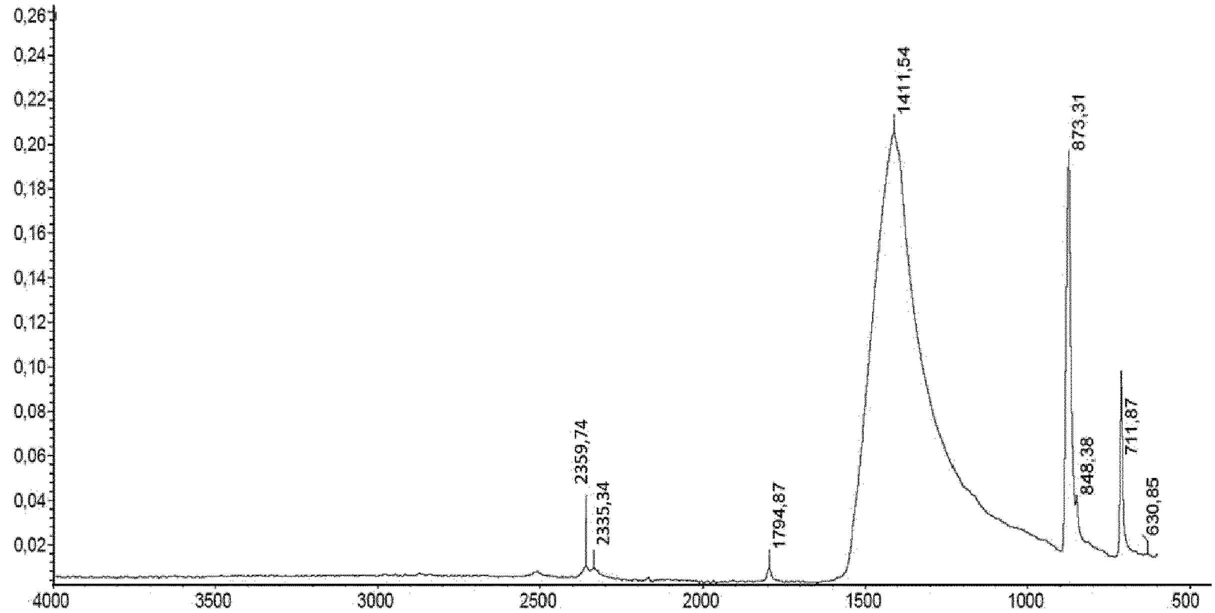


**Figure 3** FT-IR spectrum of NU1

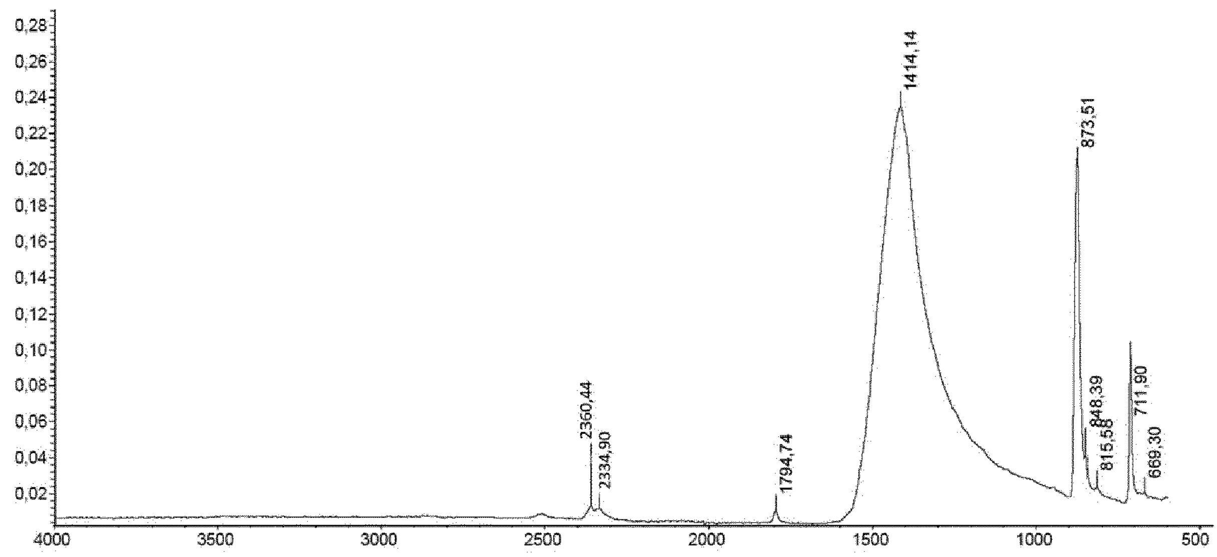


**Figure 4** FT-IR spectrum of NU2

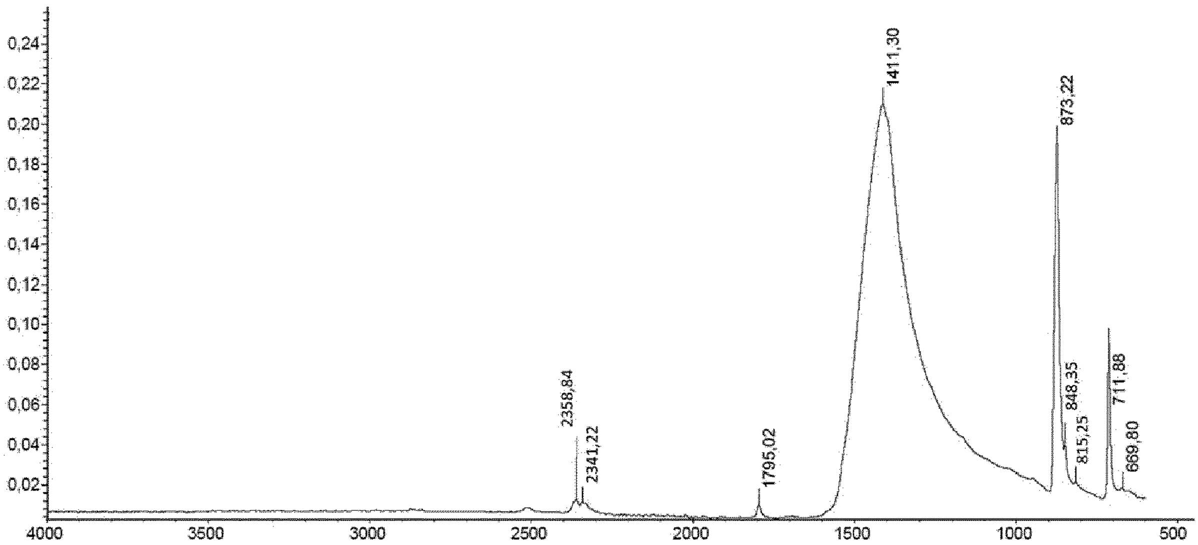


**Figure 5** FT-IR spectrum of NU3

Europäisches Patentamt
European Patent Office
Office européen des brevets

# EUROPEAN SEARCH REPORT

Application Number
EP 21 20 3992

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | EP 2 799 478 A1 (GCH TECHNOLOGY CO LTD [CN]) 5 November 2014 (2014-11-05)<br>* abstract; claims 1-4,7,8; table 5 *<br>----- | 7-15<br>1-6 | INV.<br>C07C13/20<br>C07C51/41<br>C08J3/20<br>C08K5/00<br>C08K5/098 |
| Y | DATABASE CA [Online]<br>CHEMICAL ABSTRACTS SERVICE, COLUMBUS, OHIO, US;<br>17 April 2020 (2020-04-17),<br>LI, YANLIANG ET AL: "Polypropylene composition for lamps and preparation method thereof",<br>XP002805936,<br>retrieved from STN<br>Database accession no. 2020:719382<br>* abstract * | 1-6 | |
| X<br>Y | -& CN 111 019 238 A (CHINA PETROLEUM & CHEM CORP; SINOPEC QILU CO)<br>17 April 2020 (2020-04-17)<br>* abstract; example 3 *<br>* paragraphs [0014] - [0016] *<br>----- | 7-15<br>1-6 | |
| Y | EP 2 325 245 A1 (OMYA DEVELOPMENT AG [CH])<br>25 May 2011 (2011-05-25)<br>* abstract; claims 9,14,17; tables 1a,1b *<br>* paragraphs [0082] - [0086], [0122] *<br>----- | 1-6 | TECHNICAL FIELDS SEARCHED (IPC)<br>C08J<br>C07C<br>C08K<br>C08L |

The present search report has been drawn up for all claims

1

| Place of search | Date of completion of the search | Examiner |
|---|---|---|
| Munich | 16 March 2022 | Schütte, Maya |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 21 20 3992

This annex lists the patent family members relating to the patent documents cited in the above-mentioned European search report. The members are as contained in the European Patent Office EDP file on
The European Patent Office is in no way liable for these particulars which are merely given for the purpose of information.

16-03-2022

| Patent document cited in search report | Publication date | Patent family member(s) | Publication date |
|---|---|---|---|
| EP 2799478 A1 | 05-11-2014 | CN 102558683 A | 11-07-2012 |
| | | EP 2799478 A1 | 05-11-2014 |
| | | ES 2624796 T3 | 17-07-2017 |
| | | PL 2799478 T3 | 31-08-2017 |
| | | TW 201326278 A | 01-07-2013 |
| | | US 2014364553 A1 | 11-12-2014 |
| | | WO 2013097647 A1 | 04-07-2013 |
| CN 111019238 A | 17-04-2020 | NONE | |
| EP 2325245 A1 | 25-05-2011 | AU 2010321112 A1 | 07-06-2012 |
| | | BR 112012011815 A2 | 28-07-2020 |
| | | CA 2780975 A1 | 26-05-2011 |
| | | CN 102686659 A | 19-09-2012 |
| | | CN 105602001 A | 25-05-2016 |
| | | DK 2325245 T3 | 27-10-2014 |
| | | EP 2325245 A1 | 25-05-2011 |
| | | EP 2501752 A1 | 26-09-2012 |
| | | ES 2513829 T3 | 27-10-2014 |
| | | IL 219797 A | 31-08-2015 |
| | | JP 5685263 B2 | 18-03-2015 |
| | | JP 2013511581 A | 04-04-2013 |
| | | KR 20120096008 A | 29-08-2012 |
| | | MX 341990 B | 09-09-2016 |
| | | MY 161762 A | 15-05-2017 |
| | | NZ 600044 A | 31-05-2013 |
| | | PL 2325245 T3 | 31-03-2015 |
| | | PT 2325245 E | 03-11-2014 |
| | | RU 2012125273 A | 27-12-2013 |
| | | SI 2325245 T1 | 28-11-2014 |
| | | TW 201125907 A | 01-08-2011 |
| | | UA 106897 C2 | 27-10-2014 |
| | | US 2012264866 A1 | 18-10-2012 |
| | | WO 2011061094 A1 | 26-05-2011 |
| | | ZA 201203528 B | 28-08-2013 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0177961 A2
- WO 2008074494 A1


### Non-patent literature cited in the description


- **XIAO W et al.** *J. Appl. Polym. Sci.,* 2009, vol. 111, 1076-1085
- **BUSICO, V. ; CIPULLO, R.** *Prog. Polym. Sci.,* 2001, vol. 26, 443
- **BUSICO, V. ; CIPULLO, R. ; MONACO, G. ; VACATELLO, M. ; SEGRE, A.L.** *Macromolecules,* 1997, vol. 30, 6251
- **ZHOU, Z. ; KUEMMERLE, R. ; QIU, X. ; REDWINE, D. ; CONG, R. ; TAHA, A. ; BAUGH, D. ; WINNIFORD, B.** *J. Mag. Reson.,* 2007, vol. 187, 225
- **BUSICO, V. ; CARBONNIERE, P. ; CIPULLO, R. ; PELLECCHIA, R. ; SEVERN, J. ; TALARICO, G.** *Macromol. Rapid Commun.,* 2007, vol. 28, 11289
- **RESCONI, L. ; CAVALLO, L. ; FAIT, A. ; PIEMONTESI, F.** *Chem. Rev.,* 2000, vol. 100, 1253
- **WANG, W-J. ; ZHU, S.** *Macromolecules,* 2000, vol. 33, 1157
- **CHENG, H. N.** *Macromolecules,* 1984, vol. 17, 1950
- **CHALLA F ; HERMANS PH ; WEIDINGER A.** *Makromol. Chem.,* 1962, vol. 56, 169
- **TURNER-JONES A ; AIZLEWOOD JM ; BECKETT DR.** *Makromol. Chem.,* 1974, vol. 75, 134
- **PAE KD.** *J. Polym. Sci.,* 1968, vol. 6, 657
- **OBADAL M ; CERMAK R ; STOKLASA K.** *Macromol. Rapid Commun.,* 2005, vol. 26, 1253
- *CHEMICAL ABSTRACTS,* 2305-26-2
- *CHEMICAL ABSTRACTS,* 31570-04-4
- *CHEMICAL ABSTRACTS,* 6683-19-8